# EUROPÄISCHE PATENTSCHRIFT

(11) **EP 4 205 808 B1**
(45) Veröffentlichungstag und Bekanntmachung des Hinweises auf die Patenterteilung: **29.10.2025**
(21) Anmeldenummer: 21218326.3
(22) Anmeldetag: 30.12.2021
(51) Int. Cl.: A61N 5/06, A61F 13/00

(54) **VORRICHTUNG ZUR ANTIMIKROBIELLEN WUNDBEHANDLUNG**
ANTIMICROBIAL WOUND TREATMENT DEVICE
DISPOSITIF DE TRAITEMENT ANTIMICROBIEN DES PLAIES

(43) Veröffentlichungstag der Anmeldung: 05.07.2023
(73) Patentinhaber: Paul Hartmann AG, 89522 Heidenheim (DE)
(72) Erfinder: DEIBLER, Martin, 89542 Herbrechtingen (DE); CROIZAT, Pierre, 89542 Herbrechtingen (DE)
(74) Vertreter: Paul Hartmann AG Patents & Licensing

(56) Entgegenhaltungen:
- WO-A2-2012/031282
- FR-A1- 2 986 156
- KR-A- 20080 018 360
- US-A1- 2020 101 161

## Beschreibung

Die vorliegende Erfindung betrifft eine Vorrichtung zur Therapie von Wunden zur lokalen Therapie von mikrobiellen Kontaminationen, Infektionen und Biofilmen in Wunden durch Plasmonenresonanz-induzierte thermische Mikroablation.

Mikrobielle Kontaminationen und Infektionen stellen eine Herausforderung in der Behandlung von Wunden, insbesondere in der Behandlung von chronischen Wunden, dar. Bei einer Therapie von infizierten Wunden mittels antimikrobieller Wirkstoffe können Resistenzen gegen die Wirkstoffe auftreten.

Im kosmetischen Bereich ist die Anwendung von Plasmonenresonanz bekannt. US20140371664 beschreibt eine Methode, bei der Nanopartikel auf einen kosmetischen Träger aufgebracht werden und in Hautnähe zur Plasmonenresonanz angeregt werden. Die Methode kann zur Haarentfernung und Akne-Behandlung eingesetzt werden.

Im Bereich der Wundbehandlung ist die Anwendung von Nanopartikeln zur antibakteriellen bzw. bakteriostatischen Therapie in WO2017/122224 beschrieben. Hierzu werden Silbernanopartikel mit Cellulose-Nanokristallen in einer Salbe kombiniert.

In WO2012/031282 werden Nanopartikel, welche in Fibroin eingebracht sind, zur Plasmonenresonanz angeregt. Der resultierende photothermische Effekt kann in der Thermotherapie genutzt werden.

US2020/101161 offenbart ein Verfahren zur Wundbehandlung, bei dem ein Wundverband mit antibakterieller Wirkung angelegt wird. Das Verfahren umfasst die Verwendung eines lichtempfindlichen Farbstoffs, der aktiviert werden kann, um eine einzelne oder mehrere reaktive Sauerstoffspezies zu erzeugen. Dieser Farbstoff ist auf einen Träger aufgebracht und wird mit einer transparenten Abdeckschicht bedeckt. Der Träger kann zusätzlich Nanopartikel enthalten, welche die Konzentration freier Radikale erhöhen kann.

Die der Erfindung zugrunde liegende Aufgabe besteht darin, bei einer Wundbehandlung das Risiko für Wundinfektionen zu verringern. Eine weitere Aufgabe besteht darin, die mikrobielle Besiedlung von bereits infizierten Wunden zu reduzieren. Des Weiteren soll die Ausbildung von Biofilmen in Wunden unterdrückt und bereits vorhandene Biofilme sollen möglichst wirksam eliminiert werden.

Diese Aufgaben werden durch eine Vorrichtung gemäß Anspruch 1 gelöst. Die Erfindung ermöglicht die Verbesserung der Therapie chronischer, insbesondere infizierter Wunden durch eine thermische Inaktivierung und gegebenenfalls Abtragung von Mikroorganismen und Biofilmen. Diese Hitzebehandlung kann zielgenau auf der Wundstelle oder gegebenenfalls in der Wundumgebung angewandt werden.

Die Vorrichtung umfasst eine Wundauflage und eine Lichtquelle. Optional können weitere Lichtquellen vorhanden sein. Die Wundauflage umfasst eine bei Gebrauch wundseitig angeordnete Wundkontaktschicht und eine bei Gebrauch der Wundauflage wundabseitig angeordnete oder anordenbare Abdeckschicht. Die erfindungsgemäße Wundauflage ist weiter dadurch gekennzeichnet, dass in der Wundkontaktschicht Nanopartikel vorhanden sind. Diese Nanopartikel sind geeignet zu Plasmonenresonanz angeregt zu werden. Die Lichtquelle ist wundabseitig der Abdeckschicht angeordnet oder kann wundabseitig der Abdeckschicht angeordnet werden. Die Lichtquelle ist dazu geeignet, die Nanopartikel zur Plasmonenresonanz anzuregen.

Mittels der erfindungsgemäßen Vorrichtung kann somit über einem Wundbereich ebenso wie über der in der Wundumgebung vorhandenen unversehrten Haut eines Patienten eine Plasmonenresonanz angeregt werden. Die daraus resultierende thermische Mikroablation führt zur Inaktivierung und/oder Abtragung von schädlichen Mikroorganismen oder Biofilmen in einer Wunde oder auf der Haut eines Patienten. Unter einer thermischen Mikroablation wird vorliegende thermische Inaktivierung und gegebenenfalls Abtragung von Mikroorganismen und Biofilmen verstanden.

Durch die Wechselwirkung der in der Wundkontaktschicht vorhandenen Nanopartikel mit Licht werden die Leitungselektronen in den Nanopartikeln zur Schwingung angeregt. Das Quasiteilchen, das diese Schwingung beschreibt, ist das Plasmon. Die Anregung eines Plasmons geht mit der Absorption von Licht einher und wird als Plasmonenresonanz bezeichnet.

Unter Nanopartikeln werden im Zusammenhang mit der vorliegenden Erfindung Teilchen mit einer maximalen räumlichen Ausdehnung von 1 nm bis 500 nm verstanden. Bevorzugt liegt die maximale räumliche Ausdehnung der Nanopartikel zwischen 2 nm und 200 nm, besonders bevorzugt liegt die maximale räumliche Ausdehnung von Nanopartikeln zwischen 5 nm und 150 nm, insbesondere liegt die maximale räumliche Ausdehnung von Nanopartikeln zwischen 6 nm und 100 nm.

Die maximale räumliche Ausdehnung der Nanopartikel wird im Zusammenhang mit der Erfindung vorzugsweise mittels Rasterelektronenmikroskopie bestimmt.

Die maximale Ausdehnung der Nanopartikel unterliegt einer Verteilung und kann daher von Nanopartikel zu Nanopartikel um wenige Nanometer schwanken.

Gemäß der vorliegenden Erfindung werden Nanopartikel in eine Wundkontaktschicht eingebracht oder auf eine Oberfläche der Wundkontaktschicht aufgebracht. Durch die Bestrahlung mit Licht werden diese Nanopartikel zu Schwingungen angeregt. Durch die oben beschriebene Plasmonenresonanz kann lokal im Wundbereich oder in der Wundumgebung Wärme erzeugt werden. Durch thermische Mikroablation können schädliche Mikroorganismen oder Biofilme von der Wunde oder der Haut eines Patienten entfernt werden.

Die Nanopartikel können beispielsweise in eine Silikonmatrix eingebracht sein. Auch können die Nanopartikel auf die Wundkontaktschicht aufgebracht sein, beispielsweise mittels Tauchbeschichtung oder mittels Lösungsmittelverdampfung. Bei der Lösungsmittelverdampfung werden die Nanopartikel in einem Lösungsmittel vermengt. Das Lösungsmittel wird bei reduziertem Druck entfernt, wobei die Nanopartikel ausfallen und auf der Oberfläche der Wundkontaktschicht haften.

Die Abdeckschicht soll im Wundbereich ein für die Wundheilung förderliches Milieu schaffen und verhindern, dass Verunreinigungen in die Wunde gelangen. Die Abdeckschicht kann ein integraler Teil der Wundauflage sein. Alternativ kann die Abdeckschicht als separate Komponente bereitgestellt werden und erst bei Gebrauch der Wundauflage aufgebracht werden. Die Abdeckschicht kann selbstklebend ausgeführt sein.

Die Wundauflage umfasst eine Wundkontaktschicht. Die Wundkontaktschicht kommt bei Anwendung der Wundauflage direkt auf der Haut oder Wundstelle des Patienten zum Liegen.

Die Wundkontaktschicht kann einen adhäsiven Bereich zum Befestigen der Wundauflage aufweisen.

Die Wundauflage umfasst eine Zwischenschicht. Die Zwischenschicht umfasst bevorzugt ein Flüssigkeits-absorbierendes Material, wie beispielsweise Zellulosefasern, Baumwollfasern, Alginatfasern, hydrophile synthetische Polymere oder superabsorbierende Partikel oder Fasern. Das Material kann beispielsweise in Form eines Non-Woven, Polymerschaums oder Hydrogels vorhanden sein. Vorzugsweise enthält die Zwischenschicht ein Vlies oder Airlaid, in welches superabsorbierende Partikel aus Polyacrylat eingearbeitet sind. Die Zwischenschicht ist zwischen Abdeckschicht und Wundkontaktschicht angeordnet. Es können auch mehrere Zwischenschichten, insbesondere aus unterschiedlichen Materialien, vorhanden sein.

Bei den einen oder mehreren Zwischenschichten kann es sich um eine oder mehrere Transferschichten handeln. Eine Transferschicht ermöglicht insbesondere einen unidirektionalen Transport von aus der Wunde abgegebenem Wundsekret. Bevorzugte Ausführungsformen einer solchen Transferschicht umfassen Materialien, die Kapillareffekte zulassen oder hygroskopische Eigenschaften aufweisen, wie beispielsweise Fleece, superabsorbierende Fasern oder Partikel oder perforierte Folien.

Bei den Nanopartikeln handelt es sich in einer bevorzugten Ausführungsform um Nanostäbchen. Diese weisen insbesondere eine maximale räumliche Ausdehnung von 45 nm auf. Die Nanopartikel können gleichfalls in weiteren Formen vorliegen, z.B. Kubus, Kugel, Tetrahedron, Octahedron, Plättchen oder Röhrchen. Eine mögliche Ausführungsform der Nanopartikel kann Nanoröhrchen, Nanostäbchen, Nanofasern, aber auch Nanoplättchen oder hohle geschlossene Moleküle umfassen. Beispiele für solche Ausführungsformen sind Goldnanostäbchen oder Goldnanohüllen um einen Siliziumoxidkern.

Vorzugweise umfassen die Nanopartikel metallische Ausgangssubstanzen oder metallische Salze und Oxide. Besonders bevorzugt bestehen die Nanopartikel aus den vorgenannten metallischen Ausgangssubstanzen oder metallischen Salzen und Oxiden. Gemäß einer besonders vorteilhaften Ausführungsform umfassen die Nanopartikel Gold oder Zinkoxid oder bestehen ausschließlich daraus.

Nanopartikel können auch Silber, Ruthenium, Platin, Rhodium, Osmium, Iridium, Kupfer, Zink, Nickel, Chrom, Magnesium, Eisen, Palladium, Gold, Titan, Titandioxide, Silber, Silbernitrat und weiter Silber-Derivate umfassen.

Sowohl die Abdeckschicht als auch jede der Zwischenschichten umfasst entweder einen transparenten Bereich und/oder eine Aussparung. Hierbei sind der oder die transparenten Bereiche oder Aussparungen so angeordnet, dass Licht, welches von einer wundabseitig der Abdeckschicht vorhandenen Lichtquelle in die Wundauflage eingestrahlt wird, bis zur Wundkontaktschicht gelangen kann. Die bevorzugt wundabseitig der Abdeckschicht vorhandene Lichtquelle ist vorzugsweise mit der Abdeckschicht lösbar oder unlösbar verbunden. Im Falle einer Lichtquelle, die wundabseitig lösbar mit der Abdeckschicht verbunden ist, kann es vorgesehen sein, dass die Lichtquelle wiederverwertbar ist und auf einer weiteren Wundauflage lösbar befestigt werden kann.

Die Aussparung oder das transparente Fenster in der Abdeckschich kann blickdicht verschlossen werden, solange keine Plasmonen angeregt werden. Zum Abdecken der Aussparung oder des transparenten Fensters kann eine blickdichte Folie verwendet werden. Die blickdichte Folie kann mittels adhäsiver Bereiche auf der Abdeckschicht befestigt werden. Alternativ kann die blickdichte Folie an einer Seite fest mit der Abdeckschicht verbunden sein, so dass die blickdichte Folie aufklappbar ist. Der wiederverschließbare Bereich kann dabei durch elektrostatische Wechselwirkung zwischen den Folien an der Abdeckschicht fixiert werden.

In einer anderen Ausführungsform kann der wiederverschließbare Bereich mittels geeigneter Oberflächenstrukturen des aufklappbaren Bereichs wieder verschlossen werden (Gecko Effekt).

Im Inneren der Wundauflage ist vorzugsweise keine fest in die Wundauflage integrierte Lichtquelle vorgesehen. Um die in der Wundkontaktschicht vorhandenen Plasmonen anzuregen muss deshalb die externe Lichtquelle eingesetzt werden. Das Verwenden einer externen Lichtquelle gewährt Flexibilität beispielsweise hinsichtlich Wahl der Lichtintensität oder Wellenlänge des Lichts.

Bei der Lichtquelle handelt es sich bevorzugt um eine Laser-LED oder eine LED. Es kann sich auch um eine Anordnung von mehreren Laser-LEDs oder LEDs in einem Array handeln. Falls zur Plasmonenanregung ein Laser eingesetzt wird, darf der Laser eine Flächenleistung 330 bis 350 mW/cm² für 10 s bis 100 s nicht überschreiten, sonst kann es zu Zell- und Hautschäden kommen. Es wird bevorzugt der Wellenlängenbereich 650 nm bis 940 nm im Nahinfrarotbereich und/oder 1000 nm bis 1350 nm im Infrarotbereich verwendet.

Die Wundauflage kann adhäsive Bereiche zum Befestigen einer Lichtquelle aufweisen. Die adhäsiven Bereiche können einen druckempfindlichen Klebstoff umfassen. Für die adhäsiven Bereich können beispielsweise Polyurethan-Klebstoffe oder Acrylatklebstoffe verwendet werden. Alternativ kann die Lichtquelle selbst Befestigungsmittel umfassen, die eine Fixierung wundabseitig der Abdeckschicht erlauben, so dass das von der Lichtquelle abgestrahlte Licht bis zur Wundkontaktschicht gelangen kann. Als Befestigungsmittel sind Clipbefestigungen, Schnallen, Druckknöpfe, magnetische Befestigungen und/oder Schlaufen denkbar. In einer anderen Ausführungsform kann die Lichtquelle ohne Befestigung frei platziert werden. Eine frei platzierbare Lichtquelle kann besonders bei einer stationären Wundbehandlung zum Einsatz kommen.

Die Lichtquelle, die zur Erzeugung der Plasmonenresonanz verwendet wird, strahlt Licht im Wellenlängen-Bereich zwischen 300 nm und 2000 nm aus.

Es wird der Wellenlängenbereich von 650 nm bis 940 nm im Nahinfrarotbereich und von 1000 nm bis 1350 nm im Infrarotbereich bevorzugt. Bevorzugt strahlt die Lichtquelle monochromatisches Licht aus. Bevorzugt strahlt die Lichtquelle monochromatisches Licht aus, welches auf die Größe und Form der Nanopartikel optimal abgestimmt ist, so dass Plasmonen-Resonanz auftritt. Gemäß einer anderen bevorzugten Ausführungsform wird eine multimodale Lichtquelle verwendet. Die multimodale Lichtquelle strahlt Licht mit unterschiedlichen diskreten Frequenzen ab.

Bei einer Bestrahlungsdauer von 10 s bis 100 s darf das von der Lichtquelle ausgestrahlte Licht 350 mW/cm² nicht überschreiten, da sonst beim Patienten Zell- oder Hautschäden verursacht werden können.

Als Lichtquelle eignet sich beispielsweise die Thorlabs M850L3 Mounted LED mit einer Leistung von 900 mW bei 850 nm Nominalwellenlänge.

Die Lichtquelle kann über ein integriertes Stromversorgungselement verfügen. Das Stromversorgungselement kann eine Batterie sein. In einer anderen Ausführungsform kann die Lichtquelle mittels Kabel an eine Stromquelle angeschlossen sein.

Die Stromversorgung der Lichtquelle erfolgt vorzugsweise über externe Energiequellen durch kontaktlosen Energietransfer mittels elektromagnetischer Felder. In einer Ausführungsform ist das Stromversorgungselement ein Energy Harvesting Element. Das Energy Harvesting Element nutzt bevorzugt elektromagnetische Strahlung als Energiequelle. Besonders bevorzugt sind dabei Radiowellen mit Frequenzen von 30 kHz bis zu 300 GHz. Diese Radiowellen können beispielsweise von Funktürmen, Radaranlagen, WiFi-Netzen oder Bluetooth-Verbindungen stammen. Für WiFi Netze sind Radiowellen mit Frequenzen von 2,4 GHz und einer Leistung bis 100 mW, sowie mit Frequenzen von 5 GHz und einer Leistung bis 1 W von besonderem Interesse. Auf Mobilfunknetzen/GSM beruhende Radiowellen haben nach den GSM-Standards die Frequenzen 850 MHz, 900 MHz, 1800 MHz und 1900 MHz.

In einer bevorzugten Ausführungsform umfasst die Stromversorgung der Lichtquelle ein Energiespeichermodul.

In einer anderen bevorzugten Ausführungsform wird die Lichtquelle über induktives Laden mit Energie versorgt.

### Abbildung

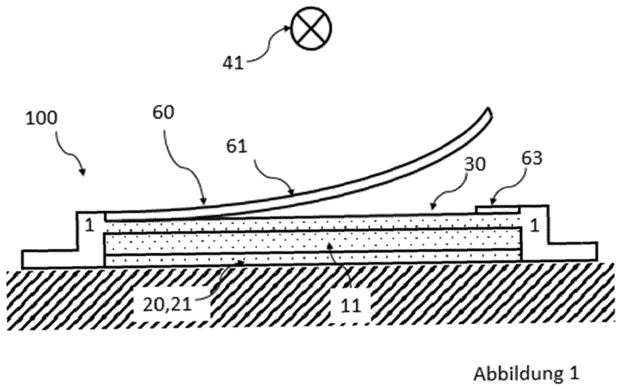

Abbildung 1 zeigt schematisch eine Ausführungsform der Vorrichtung bestehend aus Wundauflage 100 und einer externen Lichtquelle 41. Alle lichtdurchlässigen Schichten 30 sind gepunktet dargestellt.

Die Wundauflage 100 ist zur lokalen Therapie von mikrobiellen Kontaminationen, Infektionen und Biofilmen durch Plasmonenresonanz-induzierte thermische Mikroablation vorgesehen. Die Wundauflage 100 umfasst eine Abdeckschicht 1, eine Wundkontaktschicht 20 und eine absorbierende Zwischenschicht 11, wobei die Zwischenschicht 11 zwischen Abdeckschicht 1 und Wundkontaktschicht 20 angeordnet ist.

Die Wundkontaktschicht 20 enthält Nanostäbchen 21 aus Gold, wobei vorteilhaft die in der Veröffentlichung "Bacterial biofilm elimination using gold nanorod localised surface plasmon resonance generated heat" von Maria Pihlet al. (2017) (https://doi.org/10.1016/j.msec.2017.05.067.) offenbarten Goldpartikel eingesetzt werden können. Die maximale räumliche Ausdehnung der Nanostäbchen 21 beträgt 45 nm. Die Wundkontaktschicht 20 liegt bei Gebrauch der Wundauflage direkt auf der Haut und/oder der Wundstelle des Patienten auf.

Die Abdeckschicht 1 und die absorbierende Zwischenschicht 11 weisen transparente Bereiche auf. Die transparenten Bereiche sind so übereinander angeordnet, dass ein durch Abdeckschicht 1 und absorbierende Zwischenschicht 11 reichender, lichtdurchlässiger Bereich 30 entsteht. Das von der externen Lichtquelle 41 ausgestrahlte Licht durchdringt die lichtdurchlässigen Bereiche in der Abdeckschicht 1, die absorbierende Zwischenschicht 11 und trifft auf die Wundkontaktschicht 20. Dort regt das Licht die Nanostäbchen 21 zur Plasmonenresonanz an. Die daraus resultierende lokale Erhitzung führt zu einer thermischen Mikroablation und/oder einer Inaktivierung von schädlichen Mikroorganismen oder Biofilmen in einer Wunde eines Patienten.

Die externe Lichtquelle 41 ist eine LED mit 850 nm Nominalwellenlänge, sowie 3,5 mW/cm² bei 322 mW Gesamtleistung. Eine solche LED ist beispielsweise die Thorlabs LIU850A LED array, die einen Durchmesser von 38 mm hat.

Auf der Abdeckschicht 1 ist auf der wundabgewandten Seite eine blickdichte Folie 60 mit einem wiederverschließbaren Bereich 61 aufgebracht. Der wiederverschließbare Bereich 61 ist ein aufklappbarer Ausschnitt in der blickdichten Folie. Der aufklappbare Ausschnitt 61 wird durch einen adhäsiven Bereich 63 im verschlossenen Zustand fixiert.

Die Wundauflage 100 wird am Patienten aufgebracht, so dass die transparenten Bereiche 30 über der Wundfläche zum Liegen kommen. Der wiederverschließbare Bereich 61 der blickdichten Folie 60 wird aufgeklappt, so dass der lichtdurchlässige Bereich 30 freigelegt wird. Die externe Lichtquelle 41 wird über der Wundauflage 100 so ausgerichtet, dass das von der Lichtquelle 41 ausgestrahlte Licht durch den lichtdurchlässigen Bereich 30 auf die Nanopartikel 21 treffen kann. Die Lichtquelle 41 wird eingeschaltet und die Nanostäbchen 21 aus Gold werden zur Plasmonenresonanz angeregt. Die Lichtquelle 41 bleibt 5 min aktiv. Je nach Bedarf kann die Lichtquelle 41 mehrmals im Laufe der Behandlung aktiviert werden.

## Patentansprüche

1. Vorrichtung zur Verwendung bei der Inaktivierung und/oder Abtragung von schädlichen Mikroorganismen oder Biofilmen in einer Wunde eines Patienten mittels Plasmonenresonanz-induzierter thermischer Mikroablation, umfassend eine Lichtquelle (41) und eine Wundauflage (100), wobei die Wundauflage (100) eine
- eine wundseitig angeordnete Wundkontaktschicht (20), und
- eine wundabseitig angeordnete Abdeckschicht (1),
umfasst,
wobei die Wundkontaktschicht (20) Nanopartikel (21) enthält, die geeignet sind zu Plasmonenresonanz angeregt zu werden,
und wobei die wundabseitig angeordnete Abdeckschicht (1) einen transparenten Bereich (30) und/oder eine Aussparung umfasst, und
wobei die Lichtquelle (41) wundabseitig der Abdeckschicht (1) angeordnet ist oder angeordnet werden kann,
und wobei die Lichtquelle (41) dazu geeignet ist, die Nanopartikel (21) zur Plasmonenresonanz anzuregen,
und wobei die Abdeckschicht (1) einen wiederverschließbaren, lichtundurchlässigen Bereich (61) umfasst, der den transparenten Bereich (30) und/oder die Aussparung bedecken kann,
**dadurch gekennzeichnet, dass** die Wundauflage (100) mindestens eine Zwischenschicht (11) umfasst, wobei die mindestens eine Zwischenschicht (11) zwischen der Abdeckschicht (1) und der Wundkontaktschicht (20) angeordnet ist, und wobei die mindestens eine Zwischenschicht (11) einen transparenten Bereich (30) und/oder eine Aussparung umfasst, so dass ein lichtdurchlässiger Bereich zwischen der wundabseitig der Abdeckschicht (1) angeordneten oder anordenbaren Lichtquelle (41) und der Wundkontaktschicht (20) vorhanden ist, wobei die mindestens eine Zwischenschicht (11) Flüssigkeits-absorbierende Eigenschaften aufweist,
oder wobei die mindestens eine Zwischenschicht (11) eine Transferschicht ist, die einen unidirektionalen Transport von aus der Wunde abgegebenem Wundsekret ermöglicht.

2. Vorrichtung nach Anspruch 1, wobei die Lichtquelle (41) mit der Abdeckschicht (1) der Wundauflage (100) lösbar oder unlösbar verbunden ist.

3. Vorrichtung nach mindestens einem der vorgenannten Ansprüche, wobei die Wundkontaktschicht (20) einen adhäsiven Bereich aufweist.

4. Vorrichtung nach mindestens einem der vorgenannten Ansprüche, wobei es sich bei den Nanopartikeln (21) um Nanopartikel (21) aus Metall oder metallischen Salzen handelt.

5. Vorrichtung nach mindestens einem der vorgenannten Ansprüche, wobei die Nanopartikel (21) eine maximale räumliche Ausdehnung zwischen 1 nm und 500 nm, bevorzugt zwischen 2 nm und 200 nm, besonders bevorzugt zwischen 5 nm und 150 nm, insbesondere zwischen 6 nm und 100 nm haben.

6. Vorrichtung nach mindestens einem der vorgenannten Ansprüche, wobei es sich bei der Lichtquelle (41) um eine LED handelt.

7. Vorrichtung nach mindestens einem der vorgenannten Ansprüche, wobei die Lichtquelle (41) Licht mit Wellenlängen zwischen 300 nm und 2000 nm, bevorzugt 650 nm bis 940 nm oder 1000 nm bis 1350 nm, ausstrahlt.

8. Vorrichtung nach mindestens einem der vorgenannten Ansprüche, wobei die Lichtquelle (41) monochromatisches Licht ausstrahlt.

9. Vorrichtung nach mindesten einem der vorgenannten Ansprüche, wobei die Vorrichtung mindestens zwei Lichtquellen (41) umfasst.

## Claims

1. Device for use in the inactivation and/or removal of harmful microorganisms or biofilms in a wound of a patient by means of plasmon resonance-induced thermal microablation, comprising a light source (41) and a wound dressing (100), wherein the wound dressing (100) comprises
- a wound contact layer (20) arranged on the wound side, and
- a cover layer (1) arranged on the non-wound side,
wherein the wound contact layer (20) contains nanoparticles (21) which are suitable for excitation to plasmon resonance,
and wherein the cover layer (1) arranged on the non-wound side comprises a transparent region (30) and/or a cutout, and
wherein the light source (41) is arranged or can be arranged on the non-wound side of the cover layer (1),
and wherein the light source (41) is suitable for exciting the nanoparticles (21) to plasmon resonance,
and wherein the cover layer (1) comprises a resealable, light-impermeable region (61) which can cover the transparent region (30) and/or the cutout,
**characterized in that** the wound dressing (100) comprises at least one interlayer (11), wherein the at least one interlayer (11) is arranged between the cover layer (1) and the wound contact layer (20), and wherein the at least one interlayer (11) comprises a transparent region (30) and/or a cutout, so that a light-permeable region is present between the wound contact layer (20) and the light source (41) arranged or arrangeable on the non-wound side of the cover layer (1),
wherein the at least one interlayer (11) has fluid-absorbing properties,
or wherein the at least one interlayer (11) is a transfer layer which allows unidirectional transport of wound secretion released from the wound.

2. Device according to Claim 1, wherein the light source (41) is separably or inseparably connected to the cover layer (1) of the wound dressing (100).

3. Device according to at least one of the preceding claims, wherein the wound contact layer (20) has an adhesive region.

4. Device according to at least one of the preceding claims, wherein the nanoparticles (21) are nanoparticles (21) composed of metal or metallic salts.

5. Device according to at least one of the preceding claims, wherein the nanoparticles (21) have a maximum spatial extent of between 1 nm and 500 nm, preferably between 2 nm and 200 nm, more preferably between 5 nm and 150 nm, more particularly between 6 nm and 100 nm.

6. Device according to at least one of the preceding claims, wherein the light source (41) is an LED.

7. Device according to at least one of the preceding claims, wherein the light source (41) emits light with wavelengths of between 300 nm and 2000 nm, preferably 650 nm to 940 nm or 1000 nm to 1350 nm.

8. Device according to at least one of the preceding claims, wherein the light source (41) emits monochromatic light.

9. Device according to at least one of the preceding claims, wherein the device comprises at least two light sources (41).

## Revendications

1. Dispositif destiné à être utilisé lors de l'inactivation et/ou de l'enlèvement de micro-organismes ou de biofilms nuisibles dans une plaie d'un patient au moyen d'une microablation thermique induite par résonance plasmonique, comprenant une source de lumière (41) et un pansement (100), le pansement (100) comprenant
- une couche de contact (20) avec la plaie, agencée côté plaie, et
- une couche de recouvrement (1) agencée sur le côté opposé à la plaie,
la couche de contact (20) avec la plaie contenant des nanoparticules (21) qui conviennent pour être excitées en résonance plasmonique,
et la couche de recouvrement (1) agencée sur le côté opposé à la plaie comprenant une zone (30) transparente et/ou un évidement et
la source de lumière (41) étant agencée ou pouvant être agencée sur le côté opposé à la plaie de la couche de recouvrement (1),
et la source de lumière (41) convenant pour exciter les nanoparticules (21) en résonance plasmonique,
et la couche de recouvrement (1) comprenant une zone (61) non transparente à la lumière, refermable, qui peut recouvrir la zone (30) transparente et/ou l'évidement,
**caractérisé en ce que** le pansement (100) comprend au moins une couche intermédiaire (11), ladite au moins une couche intermédiaire (11) étant agencée entre la couche de recouvrement (1) et la couche de contact (20) avec la plaie et ladite au moins une couche intermédiaire (11) comprenant une zone (30) transparente et/ou un évidement de telle sorte qu'une zone transparente à la lumière est présente entre la source de lumière (41), agencée ou pouvant être agencée sur le côté opposé à la plaie de la couche de recouvrement (1), et la couche de contact (20) avec la plaie,
ladite au moins une couche intermédiaire (11) présentant des propriétés d'absorption de liquide,
ou ladite au moins une couche intermédiaire (11) étant une couche de transfert qui permet un transport unidirectionnel de sécrétions de plaie évacuées par la plaie.

2. Dispositif selon la revendication 1, la source de lumière (41) étant reliée de manière détachable ou non détachable à la couche de recouvrement (1) du pansement (100).

3. Dispositif selon au moins l'une des revendications susmentionnées, la couche de contact (20) avec la plaie présentant une zone adhésive.

4. Dispositif selon au moins l'une des revendications susmentionnées, les nanoparticules (21) étant des nanoparticules (21) en métal ou en sels métalliques.

5. Dispositif selon au moins l'une des revendications susmentionnées, les nanoparticules (21) présentant une étendue spatiale maximale entre 1 nm et 500 nm, de préférence entre 2 nm et 200 nm, de manière particulièrement préférée entre 5 nm et 150 nm, en particulier entre 6 nm et 100 nm.

6. Dispositif selon au moins l'une des revendications susmentionnées, la source de lumière (41) étant en particulier une DEL.

7. Dispositif selon au moins l'une des revendications susmentionnées, la source de lumière (41) émettant une lumière présentant des longueurs d'onde entre 300 nm et 2000 nm, de préférence de 650 nm à 940 nm ou de 1000 nm à 1350 nm.

8. Dispositif selon au moins l'une des revendications susmentionnées, la source de lumière (41) émettant une lumière monochromatique.

9. Dispositif selon au moins l'une des revendications susmentionnées, le pansement comprenant au moins deux sources de lumière (41).
